# EUROPEAN PATENT APPLICATION

(11) **EP 4 691 435 A1**
(43) Date of publication of application: **11.02.2026**
(21) Application number: 23930768.9
(22) Date of filing: 02.10.2023
(51) Int. Cl.: A61F 13/15, B65D 85/07

(54) **INDIVIDUALLY PACKAGED ABSORBENT ARTICLE AND METHOD FOR MANUFACTURING PACKAGED PRODUCT ACCOMMODATING SAME IN PACKAGE**

(30) Priority: 30.03.2023 JP 2023055946
(71) Applicant: DAIO PAPER CORPORATION, Shikokuchuo-shi Ehime 799-0492 (JP)
(72) Inventor: IWABUCHI, Haruka, Sakura-shi, Tochigi 329-1411 (JP)
(74) Representative: Epping - Hermann - Fischer
(86) International application number: PCT/JP2023/035952
(87) International publication number: WO 2024/202137

(57) **Abstract**

An individually-wrapped absorbent article includes a wrapping sheet including paper, and an absorbent article individually wrapped with the wrapping sheet. Printing is provided on an exposed surface of the wrapping sheet, which is exposed when the wrapping sheet individually wraps the absorbent article. The exposed surface has MMD of 0.050 or less before printing, and the exposed surface after the printing has a coefficient of static friction of 0.60 or greater against an exposed surface of another individually-wrapped absorbent article.

## Description

### TECHNICAL FIELD

The present invention relates to individually-wrapped absorbent articles and production methods of packaged products encasing the individually-wrapped absorbent articles.

### BACKGROUND ART

Typically, absorbent articles, such as sanitary napkins, pantyliners, incontinence pads, and the like, are provided in a state in which an absorbent article is individually encased in a package.

Patent Document 1 discloses a technology in which a mat layer having a relief pattern serving as an outermost layer is provided to a surface of a package, which encases an absorbent article, so that a friction force of the surface of the package is increased, thereby preventing load shifting during stacking of packages for display.

### RELATED-ART DOCUMENTS

### PATENT DOCUMENTS

Patent Document 1: Japanese Unexamined Patent Application Publication No. 2020-196541

### SUMMARY OF THE INVENTION

### PROBLEM TO BE SOLVED BY THE INVENTION

From reasons of hygiene during storage, convenience when carrying, and the like, absorbent articles, such as sanitary napkins, pantyliners, incontinence pads, and the like, are provided in packages as individually-wrapped absorbent articles in each of which an absorbent article is individually wrapped with a wrapping sheet. Since the wrapping sheet individually wraps an absorbent article, use of the wrapping sheet increases an amount of waste generated after use, compared with a case where a package is used. In recent years, use of a paper material to constitute a wrapping sheet has been promoted, instead of a non-woven fabric or a plastic film of the related art, in view of environmental issues.

An individually-wrapped absorbent article as described above is carried in a state in which an absorbent article is wrapped with a wrapping sheet. Thus, it may often be preferable to print on a surface of the wrapping sheet so that an appearance of the individually-wrapped absorbent article is not apparent as an absorbent article at a glance. In order to print on a wrapping sheet to enhance the design, use of paper having high smoothness and high printing operability as a wrapping sheet is considered.

On the other hand, an individually-wrapped absorbent article is encased in a package in a state in which the individually-wrapped absorbent article is stacked with other individually-wrapped absorbent articles. In the case where paper having high surface smoothness is used as a wrapping sheet, the individually-wrapped absorbent articles are likely to slip against one another when the multiple individually-wrapped absorbent articles are stacked and encased in a package, so that production efficiency may be lowered. In addition, the paper having high surface smoothness has the appearance and texture like so-called copy paper at a glance, which gives an impression as if it is cheap.

The present invention has been made in view of the above problems existing in the related art, and an object of the present invention is to provide an individually-wrapped absorbent article that can achieve both printing operability and inhibition of reduction in production efficiency even when multiple individually-wrapped absorbent articles are stacked and encased in a package.

### SOLUTION TO THE PROBLEM

In order to achieve the above object, the present invention is directed to an individually-wrapped absorbent article that includes a wrapping sheet including paper, and an absorbent article individually wrapped with the wrapping sheet. Printing is provided on an exposed surface of the wrapping sheet, which is exposed when the wrapping sheet individually wraps the absorbent article. The exposed surface has MMD (a deviation of the mean coefficient of friction) of 0.050 or less before printing, and the exposed surface after the printing has a coefficient of static friction of 0.60 or greater against an exposed surface of another individually-wrapped absorbent article.

In the present invention as configured above, MMD of the exposed surface, which is exposed when the wrapping sheet individually wraps the absorbent article, is 0.050 or less before printing, and therefore the individually-wrapped absorbent article has printing operability. In addition, a coefficient of static friction of the exposed surface after printing against an exposed surface of another individually-wrapped absorbent article is 0.60 or greater, and therefore the individually-wrapped absorbent article is less likely to slip against another individually-wrapped absorbent article when multiple individually-wrapped absorbent articles are stacked and encased in a package, thereby inhibiting reduction in production efficiency. Further, since a coefficient of static friction of the exposed surface after printing against an exposed surface of another individually-wrapped absorbent article is 0.60 or greater, the resultant appearance and texture are less likely to give an impression as if it is cheap.

Moreover, the individually-wrapped absorbent article may be configured such that a blending ratio of recycled fibers in the wrapping sheet is 20% or greater.

With the individually-wrapped absorbent article as configured above, the coefficient of static friction of the exposed surface of the individually-wrapped absorbent article against an exposed surface of another individually-wrapped absorbent article after printing becomes large.

Moreover, the individually-wrapped absorbent article may be configured such that the wrapping sheet includes fiber bundles.

With the individually-wrapped absorbent article as configured above, the coefficient of static friction of the exposed surface of the individually-wrapped absorbent article against an exposed surface of another individually-wrapped absorbent article after printing becomes also large.

Further, as a production method of a packaged product in which the individually-wrapped absorbent article is encased in a package, the production method may include: printing on the wrapping sheet; placing the absorbent article on the wrapping sheet, folding over the wrapping sheet in a manner such that a surface of the wrapping sheet, which is printed, is exposed, to wrap the absorbent article with the wrapping sheet to produce the individually-wrapped absorbent article; and stacking the individually-wrapped absorbent article with one or more individually-wrapped absorbent articles and encasing the stacked individually-wrapped absorbent articles in the package.

### EFFECTS OF THE INVENTION

According to the present invention, both printing operability and inhibition of reduction in production efficiency can be achieved even when multiple individually-wrapped absorbent articles are stacked and encased in a package.

### BRIEF DESCRIPTION OF THE DRAWINGS

[Fig. 1] Fig. 1 is a plan view illustrating one embodiment of the individually-wrapped absorbent article of the present invention.
[Fig. 2] Fig. 2 is a plan view of a developed state of the individually-wrapped absorbent article illustrated in Fig. 1.
[Fig. 3] Fig. 3 is a cross-sectional view taken along the line I-I illustrated in Fig. 1.
[Fig. 4] Fig. 4 is an enlarged view of the section II illustrated in Fig. 1.
[Fig. 5] Fig. 5 is a flowchart illustrating a production process of a packaged product in which the individually-wrapped absorbent articles illustrated in Figs. 1 to 4 are encased.
[Fig. 6] Fig. 6 is a view illustrating a state in which the individually-wrapped absorbent articles illustrated in Figs. 1 to 4 are encased in a package.

### DESCRIPTION OF EMBODIMENTS

Embodiments of the present invention will be described with reference to drawings hereinafter. In the drawings, the same referential numeral is assigned to the same or corresponding constituent component unless otherwise stated, and redundant description may be omitted.

### <Overall configuration of individually-wrapped absorbent article>

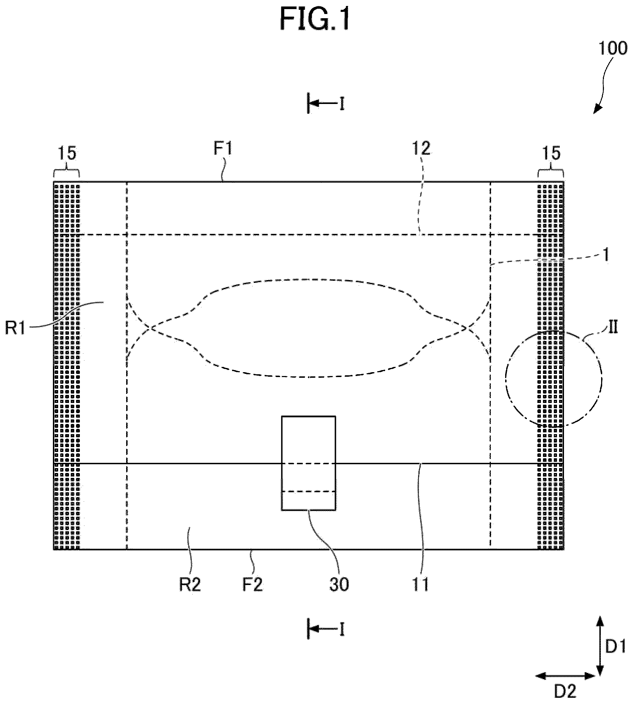

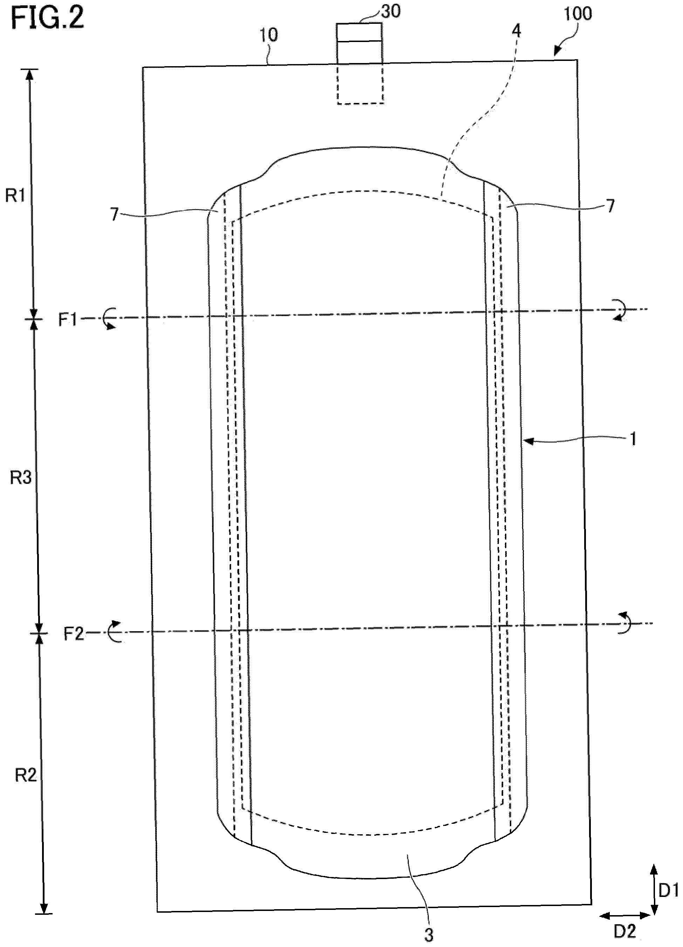

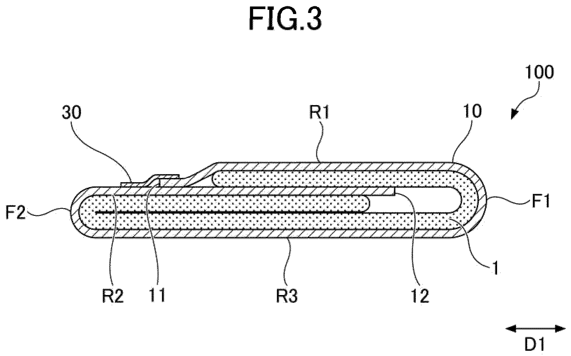

Fig. 1 is a plan view illustrating one embodiment of the individually-wrapped absorbent article of the present invention. Fig. 2 is a plan view of a developed state of the individually-wrapped absorbent article 100 illustrated in Fig. 1, and illustrates a view as viewed from the inner surface of the wrapping sheet 10 or the side of the absorbent article 1 facing the skin of a user. Moreover, Fig. 3 is a cross-sectional view taken along the line I-I illustrated in Fig. 1.

As illustrated in Figs. 1 to 3, the present embodiment is an individually-wrapped absorbent article 100 that includes a wrapping sheet 10, and an absorbent article 1 individually wrapped with the wrapping sheet 10. As illustrated in Fig. 2, the wrapping sheet 10 has, for example, a narrow and long shape in a developed state, and has a longitudinal direction (vertical direction) D1 and a lateral direction (horizontal direction) D2 orthogonal to the longitudinal direction. Moreover, the longitudinal direction D1 and the lateral direction D2 of the wrapping sheet 10 also correspond to a longitudinal direction and a lateral direction of the absorbent article 1, respectively.

### <Absorbent article>

The absorbent article 1 to be wrapped with the wrapping sheet 10 is an article having a shape that is suitable when the article is worn to face a discharge orifice of a body fluid (menstrual blood, vaginal discharge, urine, etc.), for example, a flat, narrow, and long shape. Specific examples of the absorbent article 1 includes sanitary napkins, pantyliners (vaginal discharge sheets), light incontinence pads, and the like.

The absorbent article 1 includes, for example, a fluid-permeable top sheet 3, a fluid-impermeable back sheet (not illustrated), and an absorber 4 disposed between the top sheet 3 and the back sheet, as illustrated in Fig. 2.

As the back sheet, a sheet having at least a water-shielding property, such as an olefin-based resin sheet or the like, may be used. The olefin-based resin is such as polyethylene, polypropylene, or the like. A laminate non-woven fabric, in which a non-woven fabric is laminated on a polyethylene sheet or the like, or a laminate sheet of non-woven fabrics, in which a waterproof film is inserted between the non-woven fabrics to substantially ensure a fluid impermeability, may be used as the back sheet. Further, a sheet having moisture permeability may be used as the back sheet.

As the top sheet 3, a porous or non-porous non-woven fabric, a porous plastic sheet, or the like is suitably used. As a fibrous material constituting the non-woven fabric, for example, one of, or two or more of synthetic fibers, such as olefin (e.g., polyethylene, polypropylene, etc.), polyester, polyamide, and the like, recycled fibers, such as rayon, cuprammonium rayon, and the like, blended fibers of the foregoing, and natural fibers, such as cotton and the like can be used.

The absorber 4 is not limited as long as the absorber 4 is a material that can absorb and retain a body fluid. The absorber 4 preferably includes cotton pulp and a water-absorbing polymer. As the water-absorbing polymer, a superabsorbent polymer (SAP) powder, a superabsorbent fiber (SAF), or a combination of the foregoing can be used. Examples of the pulp include chemical pulp obtained from wood, cellulose fibers, such as dissolving pulp, artificial cellulose fibers, such as rayon, acetate, and the like. The pulp may be hardwood pulp obtained from hardwood, softwood pulp obtained from softwood, or a pulp mixture of the foregoing. Moreover, the pulp may be recycled pulp that is recycled from used pulp.

A length of the absorber 4 may be from 0.5 mm to 25 mm. The absorber 4 may be configured such that a region corresponding to a body fluid discharge orifice (body-fluid-discharge-orifice corresponding region) or a region facing the intergluteal cleft behind the body-fluid-discharge-orifice corresponding region is bulged. The absorber 4 has a size and a shape that do not exceed the top sheet 3 and the back sheet. At the front and rear end edges of the absorber, the outer edges of the back sheet and the outer edges of the top sheet 3 are respectively joined by an adhesive such as a hot-melt adhesive or the like, or an adhesion method such as heat sealing or ultrasonic sealing.

As illustrated in Fig. 2, side sheets 7 may be respectively provided on the outer sides of the absorber 4 along the longitudinal direction D1 at the both edge portions of the absorber 4 in the lateral direction D2. As the side sheets 7, water-repellent non-woven fabrics or hydrophilic non-woven fabrics can be used.

Note that the absorbent article 1 is a so-called wingless type that does not have wings in the example illustrated in Figs. 1 to 3, but the absorbent article may be configured as an article having wings each extending to the side. The wings may be formed by joining the side sheet and the back sheet.

Moreover, an anti-slip adhesion portion may be provided on a back sheet side of the absorbent article 1 (the non-skin side, i.e., the side facing the underwear when worn). The anti-slip adhesion portion is provided for preventing the absorbent article 1 from being displaced from the underwear when the absorbent article 1 is attached to the underwear. The anti-slip adhesion portion is, for example, formed into multiple strips extending in the longitudinal direction D1 or the lateral direction D2.

An entire length of the absorbent article 1 can be from 140 mm to 430 mm, and a width of the absorbent article 1 (a width of a main body excluding wings if wings are provided) can be from 40 mm to 130 mm.

### <Wrapping sheet>

The wrapping sheet 10 of the present embodiment includes paper. Use of the wrapping sheet 10 made of paper can contribute to reduction in use of plastics, and to the achievement of sustainable development goals. Moreover, paper can provide unique textures, such as the appearance and feel that give gentle impressions natural materials have.

The wrapping sheet 10 includes, for example, Unryu Paper, and a design print is provided to an exposed surface of the wrapping sheet, which is exposed in a state in which the absorbent article 1 is individually wrapped with the wrapping sheet. The wrapping sheet 10 is not limited to Unryu Paper, as long as the wrapping sheet 10 satisfies the belowdescribed conditions. The conditions of the wrapping sheet 10 before and after printing will be described later.

A size of the wrapping sheet 10 depends on a size or shape of an absorbent article 1 to be wrapped. For example, a length in the longitudinal direction D1 (may be merely referred to as a length) can be from 100 mm to 450 mm, and a length in the lateral direction D2 (may be merely referred to as a width) can be from 70 mm to 250 mm in the state in which the wrapping sheet 10 is completely extended (the unfolded state). In the illustrated example, the wrapping sheet 10 has a rectangular shape in a developed state, but may have, for example, a shape, such as an oblong shape.

### <Package configuration of individually-wrapped absorbent article>

As illustrated in Figs. 1 to 3, the absorbent article 1 is wrapped by being folded together with the wrapping sheet 10, thereby forming an individually-wrapped absorbent article 100. At the time of folding, first, the absorbent article 1 is placed on the inner surface of the wrapping sheet 10 in the manner such that the back sheet side of the absorbent article 1 faces the inner surface of the wrapping sheet 10, as illustrated in Fig. 2. Then, the wrapping sheet 10 and the absorbent article 1 are folded together in the longitudinal direction D1 at the first folding line F1 and the second folding line F2 along the width direction D2. More specifically, a first region R1 including one end 11 of the wrapping sheet 10 in the longitudinal direction D1 is folded over in the longitudinal direction D1 along the first folding line F1, and a second region R2 including the other end 12 of the wrapping sheet 10 in the longitudinal direction D1 is folded over along the second folding line F2. A region of the wrapping sheet 10 between the first region R1 and the second region R2 is a third region R3. In the illustrated example, the wrapping sheet 10 is folded in a manner such that the second region R2 is folded first, and then the first region R1 is folded to overlap the outer surface of the second region R2 (Figs. 1 and 2), but the order of folding the first region R1 and the second region R2 may be reversed. After the wrapping sheet 10 and the absorbent article 1 are together folded over in three (inward tri-fold) to wrap the absorbent article 1, both edge portions in the lateral direction D2 are sealed along the longitudinal direction D1, thereby forming sealed portions 15.

Such a wrapping form of three fold or greater can be formed relatively simply, and can wrap an absorbent article 1 hygienically. In addition, the absorbent article 1 can be easily taken out. Note that the folding form is not limited to three fold, and may be four fold or more, or may be two fold.

Further, the individually-wrapped absorbent article 100 may be provided with a fastening tape 30 at a center in the lateral direction D2 in the vicinity of the edge end of the first region R1 (one end 11 of the wrapping sheet 10). As illustrated in Fig. 1, the fastening tape 30 is provided over the first region R1 and the second region R2 across one end 11 of the wrapping sheet 10. Since the fastening tape 30 is provided, a user can hold the fastening tape 30 to lift and pull the first region R1 at the time of opening the individually-wrapped absorbent article 100 so that the opening of the individually-wrapped absorbent article 100 is further facilitated. At the time of opening, the first region R1 is peeled off from the second region R2 present below the first region R1.

### <Sealed portion>

As illustrated in Fig. 1, portions of the individually-wrapped absorbent article 100 where the absorbent article 1 is not present at both edges in the lateral direction D2, are respectively sealed, thereby forming sealed portions 15. In the present embodiment, the sealed portions 15 are formed by passing through the wrapping sheet 10 between a pair of heated rolls to pressure bond the above overlapped portions of the folded wrapping sheet 10 in a thickness direction.

As illustrated in Fig. 1, the sealed portions 15 are formed, for example, along an entire length of the individually-wrapped absorbent article 100 in the longitudinal direction D1. This is preferable from the viewpoint that erroneous entry of dust or dirt, or a finger or a small object from the end edges in the lateral direction can be prevented (sealability is assured).

A range of the lateral direction D2 in which each sealed portion 15 is provided is, for example, a range of 20 mm from the end edge in the lateral direction D2. The sealed portion 15 may be formed in the entire range, or may be formed in part of the above range, for example, at a position away from the end edge in the lateral direction D2. A length (width) of each sealed portion 15 itself in the lateral direction D2 is preferably from 3 mm to 15 mm. The inner edge of the sealed portion 15 in the lateral direction D2 may be linearly extended in the longitudinal direction D1, or extended in a curved manner, for example, in a wave-like manner.

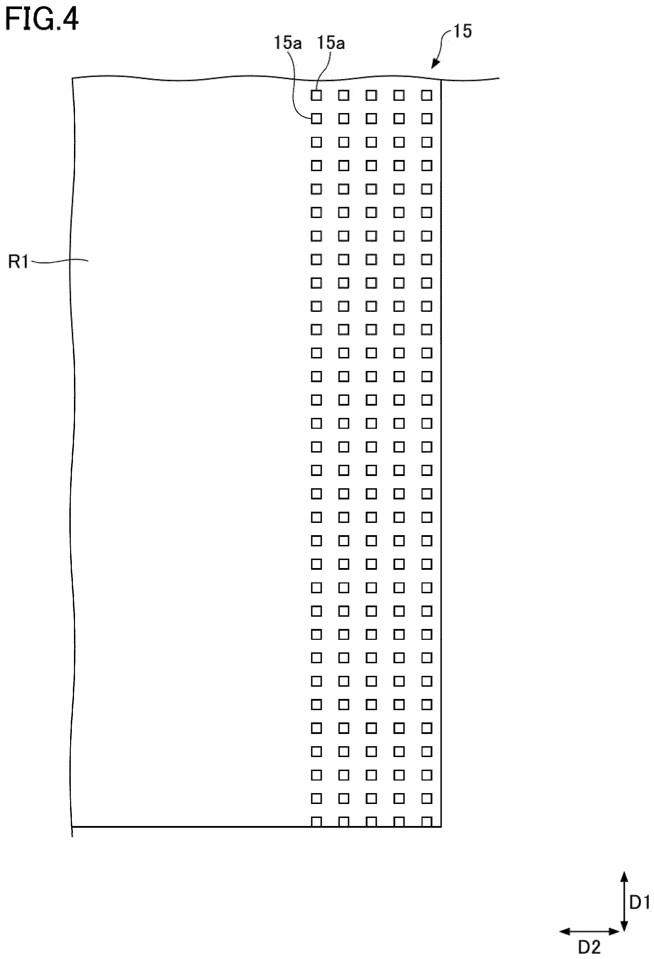

Fig. 4 is an enlarged view of the section II illustrated in Fig. 1, and illustrates a portion including the sealed portion 15.

As illustrated in Fig. 4, the sealed portion 15 includes multiple pressure-bonded portions (or bonded portions) 15a that are separated from one another in a plan view. Each pressure-bonded portion 15a is a portion in which overlapped portions of the folded wrapping sheet 10 are bonded by pressure bonding. In the illustrated example, a portion other than the pressure-bonded portions 15a is a non-bonded portion in which the overlapped portions of the folded wrapping sheet 10 are not bonded to each other.
Since the pressure-bonded portions 15a are scattered apart from one another as described above, the bonded portions of the wrapping sheet can be easily peeled off at the time of opening, and openability can be improved, for example, as compared with a case where the portions of the wrapping sheets 10 are bonded over the entire surface of the sealed portion 15. Moreover, this can prevent the both edges of the individually-wrapped absorbent article 100 from becoming excessively hard and impairing the texture.

Note that a shape of each pressure-bonded portion 15a in a plan view is a square in the example illustrated in Fig. 4, but the shape is not limited to the illustrated shape and may be, for example, a quadrangle other than a square, such as a rectangle or a parallelogram, a polygon other than a quadrangle, a circle, an ellipse, a heart shape, a star shape, a drop shape, or the like. Moreover, a size of each pressure-bonded portion 15a is, for example, a square having a side of 0.25 mm to 2.5 mm or an equivalent size having the same area as the square.

### <Production process of individually-wrapped absorbent article>

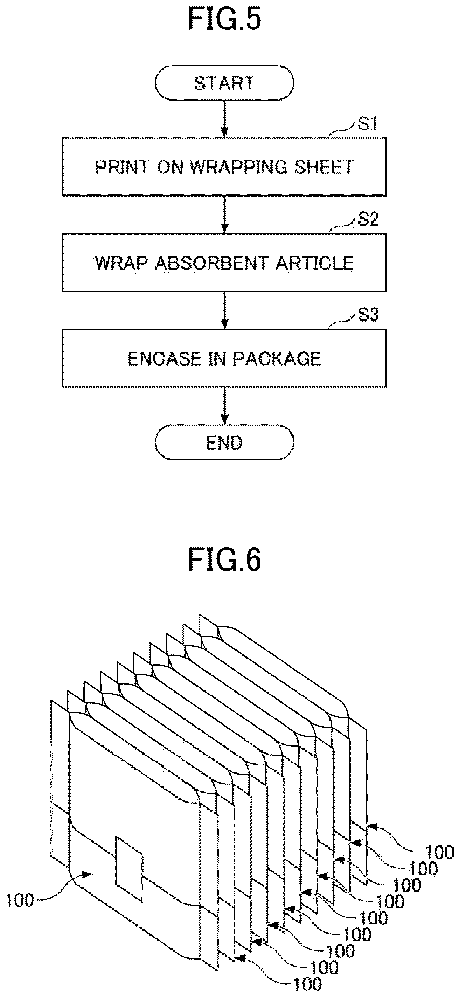

Fig. 5 is a flowchart for explaining a production process of a packaged product in which the individually-wrapped absorbent articles 100 each illustrated in Figs. 1 to 4 are encased in a package.

In the case where the individually-wrapped absorbent article 100 as configured above is produced, first, printing is performed on the wrapping sheet 10 in step S1. Note that printing of the wrapping sheet 10 is performed on an exposed surface of the wrapping sheet 10, which is exposed when the wrapping sheet 10 individually wraps the absorbent article 1.

When printing is performed on the wrapping sheet 10 to enhance the design of the individually-wrapped absorbent article 100 as described above, use of paper having high printing operability as the wrapping sheet 10 is considered. It is assumed that use of smooth paper having high surface smoothness enhances printing operability.

Therefore, various types of paper that are considered to be usable as the wrapping sheet 10 were subjected to the measurement of a deviation of the mean coefficient of friction (MMD) as an index for indicating the smoothness of the paper surface, to thereby conduct an experiment for examining whether or not printing operability was high. In this experiment, MMD was measured by a KES friction tester. As the measurement conditions, a probe wound with a piano wire was used as a probe, and a surface of a sample was scanned with the probe over the distance of 30 mm at the speed of 1 mm/sec while applying a load of 50 g.

**[Table 1]**

| Sample | Ex. 1 | Ex. 2 | Ex. 3 | Ex. 4 | Ex. 5 | Ex. 6 | Comp. Ex. 1 |
|---|---|---|---|---|---|---|---|
| Paper type | Rayon PM | Rayon PM | Unryu Paper | Unryu Paper | Kin Shachihoko | Kin Shachihoko | Crepe paper |
| Basis weight | 14 gsm | 14 gsm | 17 gsm | 17 gsm | 24.4 gsm | 24.4 gsm | 18 gsm |
| Measured surface | Smooth surface | Rough surface | Smooth surface | Rough surface | Smooth surface | Rough surface | Smooth surface |
| MMD (deviation of mean coefficient of friction) | 0.011 | 0.017 | 0.014 | 0.021 | 0.008 | 0.013 | 0.052 |
| Printing operability | A | A | A | A | A | A | B |

As presented in Table 1, as Example 1, a smooth surface of Rayon PM (produced by Marubishi Paper Tec. Corporation) having a basis weight of 14 gsm was measured. As Example 2, moreover, a rough surface of Rayon PM (produced by Marubishi Paper Tec. Corporation) having a basis weight of 14 gsm was measured. Further, as Example 3, a smooth surface of Unryu Paper (produced by Marubishi Paper Tec. Corporation) having a basis weight of 17 gsm was measured. Further, as Example 4, a rough surface of Unryu Paper (produced by Marubishi Paper Tec. Corporation) having a basis weight of 17 gsm was measured. Further, as Example 5, a smooth surface of Kin Shachihoko Paper (produced by Daio Paper Corporation) having a basis weight of 24.4 gsm was measured. Further, as Example 6, a rough surface of Kin Shachihoko Paper (produced by Daio Paper Corporation) having a basis weight of 24.4 gsm was measured. Further, as Comparative Example, a smooth surface of crepe paper (produced by Daio Paper Corporation) having a basis weight of 18 gsm was measured. Note that Rayon PM is paper including 15% of rayon, and 85% of pulp.

As a result, MMD in Comparative Example 1 was 0.052. Moreover, MMD in Example 1 was 0.011, and MMD in Example 2 was 0.017. Further, MMD in Example 3 was 0.014, and MMD in Example 4 was 0.021. Further, MMD in Example 5 was 0.008, and MMD in Example 6 was 0.013. Note that the paper in Comparative Example 1 had low smoothness because crepe processing was performed on the surface of the paper.

In Table 1, as the printing operability, "B" was given to the case where the ink was not uniformly placed on the printed surface, and white-missing portions were left, and "A" was given to the case where there were no missing portions in the printed area. As presented in Table 1, blank portions were formed in the printed area in Comparative Example 1, and no blank portions were formed in the printed area in Examples 1 to 6. Further, creases were likely to form on the surface of the paper after printing in Comparative Example 1, and it was assumed that the creases caused formation of the blank portions in the printed area.

It has been found from the above results that paper having MMD of 0.050 or less as measured on the surface of the paper before printing has printing operability as the wrapping sheet 10, and use of such paper can improve the design by printing on the surface of the wrapping sheet 10.

After printing is performed on the wrapping sheet 10, the absorbent article 1 is placed on the wrapping sheet 10, and the wrapping sheet 10 is folded over in three (inward tri-fold) together with the absorbent article 1 in a manner such that the printed surface is exposed, thereby wrapping the absorbent article 1 with the wrapping sheet 10.

Thereafter, both edges of the individually-wrapped absorbent article 100 in the lateral direction D2 are sealed along the longitudinal direction D1 to form sealed portions 15, respectively, in step S2.

Note that the process of printing on the wrapping sheet 10, the process of wrapping the absorbent article 1 with the wrapping sheet 10, and the process of forming the sealed portions 15 may be performed in the state in which the wrapping sheet 10 is in the elongated state. In this case, after forming the sealed portions 15, the wrapping sheet 10 is cut into a single piece to form the individually-wrapped absorbent article 100.

The individually-wrapped absorbent articles 100 each produced in the manner described above are encased in a package in the sequential step S3, and a packaged product in which the individually-wrapped absorbent articles 100 are encased in the package is completed (step S3).

Fig. 6 is a view illustrating a state in which the individually-wrapped absorbent articles 100 each illustrated in Figs. 1 to 4 are encased in a package.

As illustrated in Fig. 6, the individually-wrapped absorbent articles 100 each produced in the above-described manner are encased in a package in a state in which the multiple individually-wrapped absorbent articles 100 are stacked. In the case where the paper having high smoothness and high printing operability is used as the wrapping sheet 10, the individually-wrapped absorbent articles 100 are likely to slip against one another. Therefore, the stacked individually-wrapped absorbent articles 100 may come apart on the conveying line for conveying the individually-wrapped absorbent articles 100. The stacked individually-wrapped absorbent articles 100 are particularly likely to come apart in the direction of fibers of the paper. The above-described encasing of the individually-wrapped absorbent articles 100 in a package may be automatically performed on the conveying line. If the stacked individually-wrapped absorbent articles 100 come apart, therefore, the dispersed individually-wrapped absorbent articles 100 are manually encased in a package, which lowers production efficiency.

Therefore, various types of paper that are considered to be usable as the wrapping sheet 10 were subjected to the measurement of the coefficient of static friction of the paper after printing against another paper, to thereby conduct an experiment for examining whether or not individually-wrapped absorbent articles come apart when the individually-wrapped absorbent articles were inserted into a package. In the present experiment, the coefficient of static friction was measured by a tribometer. As the measurement conditions, a paper piece of 2 cm × 2 cm was moved against another paper piece of 2 cm × 2 cm over the distance of 50 mm at the speed of 43 mm/sec while applying a load of 70 g.

**[Table 2]**

| Sample | Comp. Ex. 1 | Comp. Ex. 2 | Ex. 1 |
|---|---|---|---|
| Paper type | Rayon PM | Unryu Paper | Unryu Paper |
| Basis weight | 14 gsm | 17 gsm | 17 gsm |
| Measured suface | Rough surface | Smooth surface | Rouh surface |
| Coefficient of static friction | 0.577 | 0.562 | 0.603 |
| Dispersion when inserted in package | B | B | A |

As presented in Table 2, as Comparative Example 1, a rough surface of Rayon PM having a basis weight of 14 gsm was measured. Further, as Comparative Example 2, a smooth surface of Unryu Paper having a basis weight of 17 gsm was measured. Further, as Example 1, a rough surface of Unryu Paper having a basis weight of 17 gsm was measured.

As a result, the coefficient of static friction in Comparative Example 1 was 0.577, and the coefficient of static friction in Comparative Example 2 was 0.562. On the other hand, the coefficient of static friction in Example 1 was 0.603.

In Table 2, "B" was given to the case where the individually-wrapped absorbent articles came apart when the individually-wrapped absorbent articles were inserted into the package, and "A" was given to the case where the individually-wrapped absorbent articles did not come apart. As presented in Table 2, the individually-wrapped absorbent articles came apart in Comparative Examples 1 and 2, and the individually-wrapped absorbent articles did not come apart in Example 1.

It has been found from the above results that when the paper having the coefficient of static friction of 0.60 or greater as measured on the exposed surface of the paper after printing against another paper is used as the wrapping sheet 10, the stacked individually-wrapped absorbent articles 100 are less likely to come apart when the multiple individually-wrapped absorbent articles 100 are encased in a package in the stacked state. Thus, reduction in production efficiency can be avoided when the individually-wrapped absorbent articles 100 are encased in a package. Moreover, an appearance and texture of the individually-wrapped absorbent article 100 are less likely to give an impression as if it is cheap.

Here, a relationship between the slipperiness of the individually-wrapped absorbent article 100 against another individually-wrapped absorbent article 100 and a blending ratio of regenerated fibers will be described.

Various types of paper that are considered to be usable as the wrapping sheet 10 were subjected to the measurement of the coefficient of static friction after printing against another paper, to thereby conduct an experiment for examining how the blending ratio of the regenerated fibers affects dispersion of the individually-wrapped absorbent articles when the individually-wrapped absorbent articles are inserted into a package. In the present experiment, the coefficient of static friction was measured by a tribometer. As the measurement conditions, a paper piece of 2 cm × 2 cm was moved against another paper piece of 2 cm × 2 cm over the distance of 50 mm at the speed of 43 mm/sec while applying a load of 70 g.

**[Table 3]**

| Sample | Comp. Ex. 1 | Ex. 1 |
|---|---|---|
| Paper type | Rayon PM | Unryu Paper |
| Basis weight | 14 gsm | 17 gsm |
| Measured surface | Rough surface | Rough surface |
| Coefficient of static friction | 0.577 | 0.603 |
| Ratio of regenerated fibers (rayon) | 15% | 20% to 25% |
| Dispersion when inserted in package | B | A |

As presented in Table 3, as Comparative Example 1, a rough surface of Rayon PM having a basis weight of 14 gsm was measured. Further, as Example 1, a rough surface of Unryu Paper having a basis weight of 17 gsm was measured. In the experiment, the blending ratio of the regenerated fibers was 15% in Comparative Example 1, and the blending ratio of the regenerated fibers was from 20% to 25% in Example 1.

As a result, the coefficient of static friction was 0.577 in Comparative Example 1. On the other hand, the coefficient of static friction was 0.603 in Example 1.

In Table 3, "B" was given to the case where the individually-wrapped absorbent articles came apart when the individually-wrapped absorbent articles were inserted into the package, and "A" was given to the case where the individually-wrapped absorbent articles did not come apart. As presented in Table 3, the rough surface of Rayon PM of Comparative Example 1 caused the individually-wrapped absorbent articles to come apart, and the rough surface of Unryu Paper of Example 1 did not cause the individually-wrapped absorbent articles to come apart.

It has been found from the above results that when paper in which the blending ratio of the regenerated fibers is 20% or greater is used as the wrapping sheet 10, the stacked individually-wrapped absorbent articles 100 are less likely to come apart by slipping when the multiple individually-wrapped absorbent article 100 are encased in a package in the stacked state. Thus, reduction in production efficiency can be avoided when the individually-wrapped absorbent articles 100 are encased in a package.

Here, a relationship between the slipperiness of the individually-wrapped absorbent article 100 against another individually-wrapped absorbent article 100 and fiber bundles in paper will be described.

As described above, Unryu Paper has a large coefficient of static friction against another piece of Unryu Paper, and is less likely to slip. Unryu Paper includes fiber bundles each formed by weaving rayon fibers into a twisted bundle so that Unryu Paper has large surface irregularities.

As described above, the paper including the fiber bundles has a large coefficient of static friction because such paper has large surface irregularities. It has been found from the above that, not being limited to Unryu Paper, use of the paper including the fiber bundles as the wrapping sheet 10 can make the stacked individually-wrapped absorbent articles 100 to be less likely to come apart when the individually-wrapped absorbent articles 100 are stacked and encased in a package after printing.

Although the above-described embodiment has explained that the above effects are exhibited particularly by Unryu Paper, the wrapping sheet achieving the effects is not limited to Unryu Paper. The effects of the present invention can be obtained as long as MMD of the exposed surface before printing is 0.050 or less, and the coefficient of static friction of the exposed surface of the individually-wrapped absorbent article against another exposed surface of the individually-wrapped absorbent article after printing is 0.60 or greater.

Although the present invention has been described through the embodiments above, the present invention is not limited to the above embodiments. The above embodiments can be varied by changing, modifying, replacing, adding, deleting, combining, or the like in various manners within the scope described in the claims, and such embodiments also belong within the technical scope of the present invention. Moreover, the above-described constituent components can be arbitrarily combined.

This international application claims priority based on the Japanese patent application 2023-055946 filed on March 30, 2023, the entire contents of which are incorporated herein by reference.

### DESCRIPTION OF THE REFERENCE NUMERALS

1 absorbent article
3 top sheet
4 absorber
7 side sheet
10 wrapping sheet
11 one end in the longitudinal direction
12 the other end in the longitudinal direction
15 sealed portion
15a pressure-bonded portion
30 fastening tape
100 individually-wrapped absorbent article
D1 longitudinal direction of wrapping sheet
D2 lateral direction of wrapping sheet
F1 first folding line
F2 second folding line
R1 first region
R2 second region
R3 third region

## Claims

1. An individually-wrapped absorbent article, comprising:
a wrapping sheet including paper; and
an absorbent article individually wrapped with the wrapping sheet,
wherein printing is provided on an exposed surface of the wrapping sheet, which is exposed when the wrapping sheet individually wraps the absorbent article, and
wherein the exposed surface has MMD of 0.050 or less before printing, and the exposed surface after the printing has a coefficient of static friction of 0.60 or greater against an exposed surface of another individually-wrapped absorbent article.

2. The individually-wrapped absorbent article according to claim 1,
wherein a blending ratio of recycled fibers in the wrapping sheet is 20% or greater.

3. The individually-wrapped absorbent article according to claim 1,
wherein the wrapping sheet includes fiber bundles.

4. A production method for a packaged product in which the individually-wrapped absorbent article of any one of claims 1 to 3 is encased in a package, the production method comprising:
printing on the wrapping sheet;
placing the absorbent article on the wrapping sheet, folding over the wrapping sheet in a manner such that a surface of the wrapping sheet, which is printed, is exposed, to wrap the absorbent article with the wrapping sheet to produce the individually-wrapped absorbent article; and
stacking the individually-wrapped absorbent article with one or more individually-wrapped absorbent articles and encasing the stacked individually-wrapped absorbent articles in the package.
